# EUROPEAN PATENT APPLICATION

(11) **EP 4 796 038 A1**
(43) Date of publication of application: **26.08.2026**
(21) Application number: 25159736.5
(22) Date of filing: 24.02.2025
(51) Int. Cl.: A61B 5/361, A61B 5/363, A61B 5/366, A61B 5/00, G16H 50/20, G16H 50/30

(54) **PATIENT MONITORING METHOD AND DEVICE**

(71) Applicant: Koninklijke Philips N.V., 5656 AE Eindhoven (NL)
(72) Inventor: BONOMI, Alberto Giovanni, 5656 AG Eindhoven (NL)
(74) Representative: Philips Intellectual Property & Standards

(57) **Abstract**

The present invention relates to a patient monitoring method and device (10) for monitoring a patient. The method comprises obtaining one or more physiological signals of the patient, determining an event risk of a future health adverse event of the patient based on one or more of the obtained physiological signals, real-time monitoring of a current health adverse event of the patient based on one or more of the obtained physiological signals using a monitoring algorithm having one or more adjustable configuration parameters, and adjusting one or more of the adjustable configuration parameters of the monitoring algorithm based on the determined event risk.

## Description

### FIELD OF THE INVENTION

The present invention relates to a patient monitoring method and device for monitoring a patient, e.g. a subject/person in a hospital or care center or at home.

### BACKGROUND OF THE INVENTION

Arrhythmia detection in the ICU is crucial for patient safety but presents significant challenges due to the high frequency of false alarms. The accuracy of arrhythmia detection systems is often compromised by signal noise and patient movement, leading to alarm fatigue among healthcare staff. Alarm fatigue occurs when the constant presence of alarms desensitizes caregivers, resulting in slower response or missed alarms, which can have serious consequences on patient outcomes.

Similar problems may generally appear in patient monitoring where a patient in monitored for any or certain adverse events and where a tradeoff may need to be made between sensitivity and/or accuracy of the monitoring and the number of accepted false alarms that may lead to alarm fatigue.

### SUMMARY OF THE INVENTION

It is an object of the present invention to further improve a patient monitoring method and device, particularly for real-time monitoring, to generate less false alarms while maintaining or even increasing the sensitivity and/or accuracy of the detection of adverse events.

In a first aspect of the present invention a patient monitoring method for monitoring a patient is presented, the method comprising:
- obtaining one or more physiological signals of the patient;
- determining an event risk of a future health adverse event of the patient based on one or more of the obtained physiological signals;
- real-time monitoring of a current health adverse event of the patient based on one or more of the obtained physiological signals using a monitoring algorithm having one or more adjustable configuration parameters; and
- adjusting one or more of the adjustable configuration parameters of the monitoring algorithm based on the determined event risk.

In a further aspect of the present invention a corresponding patient monitoring device for monitoring a patient is presented.

**In** yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed device, computer program and medium have similar and/or identical preferred embodiments as the claimed system, in particular as defined in the dependent claims and as disclosed herein.

Real-time monitoring algorithms should be computationally efficient to run on embedded systems and respond in a few seconds to the onset of adverse events. This may make them difficult to adapt and use for future prediction of critical health adverse events like arrhythmia. Instead, prediction algorithms typically need large and rich input data to process physiological waveforms and contextual clues provided by longitudinal observations. These algorithms, e.g. AI models, produce an output with a delay which is not suitable for real-time applications and are often designed to run on remote/cloud computing systems instead of local CPU within monitoring systems.

The present invention is based on the idea of leveraging on the output from a predictive algorithm that can indicate the time interval during which it is more likely for an adverse event to occur, i.e., that determines an event risk of a future health adverse event of the patient. The output of this prediction is used to modify one or more configuration parameters of the monitoring algorithm that is used for real-time monitoring for the occurrence of an adverse event, which may be a particular adverse event like or a critical arrhythmia event or any critical health adverse event. In this way, algorithms for real-time adverse event detection generate less false alarms while boosting their sensitivity for adverse events.

Generally, any kind of algorithms or models may be used for determining an event risk of a future health adverse event and for real-time monitoring of a current health adverse event. The selection of which algorithms or models to use may particularly depend on the kind of application and monitoring, i.e., which patient and/or which health adverse event shall be monitored.

In an exemplary embodiment, a Philips-proprietary algorithm known as ST/AR may be used that is a real-time patient monitoring algorithm capable to detect arrhythmias, measure QT-interval and ST-segment characteristics of the ECG and generate alarms for life-threatening conditions. The ST/AR arrhythmia monitoring algorithm is designed to process one or two simultaneous channels of surface ECG signals for detecting changes in the ECG rhythm while offering continuous patient surveillance and alarm generation. Such a monitoring algorithm is highly configurable to allow optimization of clinical workflows and make effective use of monitoring for identification of severe cardiac events.

The following parameters are typically configurable on hospital monitors to optimize cardiac arrhythmia detection and alarming: the sensitivity for beat detection, the duration of an adverse cardiac event before being detected as a serious event, and the characteristics of the real-time algorithm output to identify an arrhythmia, etc.

In another embodiment, a prediction algorithm (e.g. using a large neural network) may be used for future risk prediction by processing waveform data from a patient in stable condition to identify risk for future arrhythmia (or other health adverse events) by extracting hidden information from one or more raw physiological signals. A deep convolutional neural network may be used to identify risk for future ventricular tachycardia (or other health adverse events) in the next hours. Other AI models have been designed to identify patients at risk for future ischemic heart disease, atrial fibrillation, and syncope. These algorithms can identify hidden and complex patterns which can be related to specific health outcomes by processing relatively large data to produce these innovative risk scores. On the contrary, real-time monitoring algorithm like ST/AR are designed to process short data strips and rapidly produce an interpretation of the current patient status and alarm the clinical staff.

It shall be noted that the physiological signal(s) used for risk prediction and real-time monitoring can be the same but may also be different. For example, a future risk for ventricular tachycardia may be derived from a heart rate variation (HRV) signal, while real-time detection of the same event which generates alarms may be derived from an ECG.

According to a preferred embodiment, one or more of the adjustable configuration parameters of the monitoring algorithm are adjusted to increase sensitivity of the monitoring algorithm if the determined event risk indicates an increased risk of a future health adverse event and/or to decrease sensitivity and increase precision of the monitoring algorithm if the determined event risk indicates a reduced risk of a future health adverse event. This supports the desired effect of increasing sensitivity and/or diagnostic yield and at the same time reducing the number of false alarms to avoid alarm fatigue among caregivers of the patient.

In an embodiment it is determined if the determined event risk indicates an increased or decreased risk of a future health adverse event based on one or more predetermined or settable risk thresholds or risk scores. The risk threshold(s) or risk score(s) may be fixed and preset, e.g. by the designer or user of the monitoring device and method. In other embodiment, they may be adjustable, i.e., can be set for each particular patient or patient group or depending on the kind of health adverse event(s) to be detected and monitored.

The adjustable configuration parameters of the monitoring algorithm may include one or more of:
- amplitude of an event detection threshold;
- minimum event duration;
- one or more of sequence length, periodicity, number, interval of one or more event-related features in the one or more obtained physiological signals;
- one or more alarm latching properties;
- number and/or types of template families used for classification of one or more event-related features;
- noise tolerance level; and
- number of physiological signals analyzed;
- processing window (duration, time length) of physiological signal analyzed, and
- operative point of a machine learning model configured to process one or more real-time signals physiological signals.

For instance, the following adjustments of exemplary configuration parameters may be made to increase sensitivity: increase a threshold used for the amplitude to reduce sensitivity for tachycardic events or the opposite for bradycardic events; shorten an event duration; decrease the number of independent concordant detected events in different physiological parameters or channels; reduce or eliminate latching; increase the number to template families; increase noise threshold; increase the number of signals; reduce the processing window of a physiological signal analyzed.

Generally, any kind of physiological signals may be used. The one or more physiological signals may, e.g., include one or more of an ECG in one or more ECG channels, an inter-beat interval signal, a photoplethysmogram, blood pressure, SpO2, respiration rate, and body temperature. It shall be understood that the expression "physiological signal" shall not only be understood as a directly measured signal (e.g. ECG, blood pressure, etc.), but includes derived signals that are derived from one or more directly measured signals (e.g. HRV, HR, SpO2, etc.). Further, any kinds of vital signs shall be understood as physiological signal.

In an advantageous embodiment, the obtained physiological signal is an ECG and the adjustable configuration parameters of the monitoring algorithm include one or more of:
- amplitude of QRS detection threshold;
- minimum event duration for arrhythmia-like ventricular tachycardia;
- sequence length of beats labelled as ventricular beats indicating a ventricular tachycardia;
- one or more alarm latching properties;
- number and/or types of template families used for classification of beats with new and different morphologies;
- noise tolerance level; and
- number of ECG waveform channels analysed.

According to another embodiment, patient information, in particular one or more of age, weight, size, gender, ethnicity, BMI, medication, medical treatment, and medical history, is used in addition to the one or more obtained physiological signals to determine the event risk and/or to adjust one or more of the adjustable configuration parameters. For instance, a patient's medical history may have an influence on the desired sensitivity of the real-time monitoring and on the risk prediction (e.g. a patient who suffered from myocardial infarction in the past may have a higher risk of suffering from cardiac events in the future). Further, a patient having a high BMI may have a higher risk of future adverse events than a patient having a low BMI.

Preferably, an AI-based prediction algorithm is used to determine the event risk. For instance, an AI-based prediction model may be used, such as a U-net or a ResNet, configured to output a binary probability value indicating the risk of future adverse events in a pre-determined window of time. Input to the ResNet or U-net may be the raw waveform from one or more physiological waveforms such as ECG and/or contextual information like HR or HRV over time and static parameters like demographics and patient treatment and characteristics.

According to another embodiment, in case of a reduced event risk one or more of the adjustable configuration parameters of the monitoring algorithm are adjusted to use default configuration parameters, or to use previously used configuration parameters that have been previously used in case of a reduced event risk, or to maintain the currently used configuration parameters. Thus, "adjusting one or more of the adjustable configuration parameters of the monitoring algorithm" shall be understood such that in certain situations not necessarily any configuration parameter(s) is (are) changed, but depending on the situation (in particular the future event risk) one or more (or even all) configuration parameters may be kept unchanged or may be set to default values or values that have been previously used. In an embodiment, a change of one or more (or even all) configuration parameters is mainly made in case the event risk increases, e.g. if it increases by a certain amount or percentage or above an upper risk threshold or risk score level. In case of a subsequent reduction of the event risk these changes may then be reversed, e.g. if it decreases by a certain amount or percentage or falls below a lower risk threshold or risk score level.

In another embodiment, for determining the event risk one or more portions of the one or more obtained physiological signals of a duration in the range between 5 minutes and 10 hours, in particular the last minutes or hours of the one or more obtained physiological signals, are analyzed, and for real-time monitoring of the current health adverse event one or more portions of the one or more obtained physiological signals of a duration in the range between 10 seconds and 10 minutes, in particular the last seconds or minutes of the one or more obtained physiological signals, are analyzed. The particular durations of the one or more obtained physiological signals used for the respective analyses may be preset and fixed or may be set or modified by the user. These durations may depend on one or more of the particular application, physiological signal, adverse events to be detected and monitored, the particular patient, patient information, etc.

The patient monitoring method may further comprise the step of generating and/or issuing one or more of:
- an alarm in case of detection of a current health adverse event;
- the one or more obtained physiological signals;
- the results of the detection of a current health adverse event and/or the determination of the event risk;
- a notification of the adjustment of one or more configuration parameters; and
- the one or more adjusted configuration parameters.
Such actions may be preset or may be set by the user, depending on the same criteria as mentioned above for setting the durations of physiological signal(s) used for the different analyses.

The patient monitoring device according to the present invention may be a typical patient monitor, as typically used in a hospital as bedside monitor. Alternatively, the patient monitoring device may be implemented as computer, table, smartphone or processor or another device. The processing steps may be carried out by a processor of the patient monitoring device but may alternatively be carried out by a cloud computer or a medical server or data center in a hospital.

The patient monitoring device may further comprise a user interface for inputting and/or outputting information, in particular including one or more of a display, touchscreen, loudspeaker, computer monitor, screen of a smartphone and/or tablet.

### BRIEF DESCRIPTION OF THE DRAWINGS

These and other aspects of the invention will be apparent from and elucidated with reference to the embodiment(s) described hereinafter. In the following drawings:
Fig. 1 shows a diagram illustrating the difference between real-time monitoring and event risk prediction in a typical ECG monitoring scenario.
Fig. 2 shows a schematic diagram of an embodiment of a patient monitoring device according to the present invention.
Fig. 3 shows a flowchart of a first embodiment of a patient monitoring method according to the present invention.
Fig. 4 shows a flowchart of a method for risk prediction according to an embodiment of the present invention.
Fig. 5 shows a flowchart of a method for adjusting configuration parameters according to an embodiment of the present invention.
Fig. 6 shows a flowchart of a second embodiment of a patient monitoring method according to the present invention.
Fig. 7 shows a diagram illustrating the expected alarming behavior of the disclosed patient monitoring device.

### DETAILED DESCRIPTION OF EMBODIMENTS

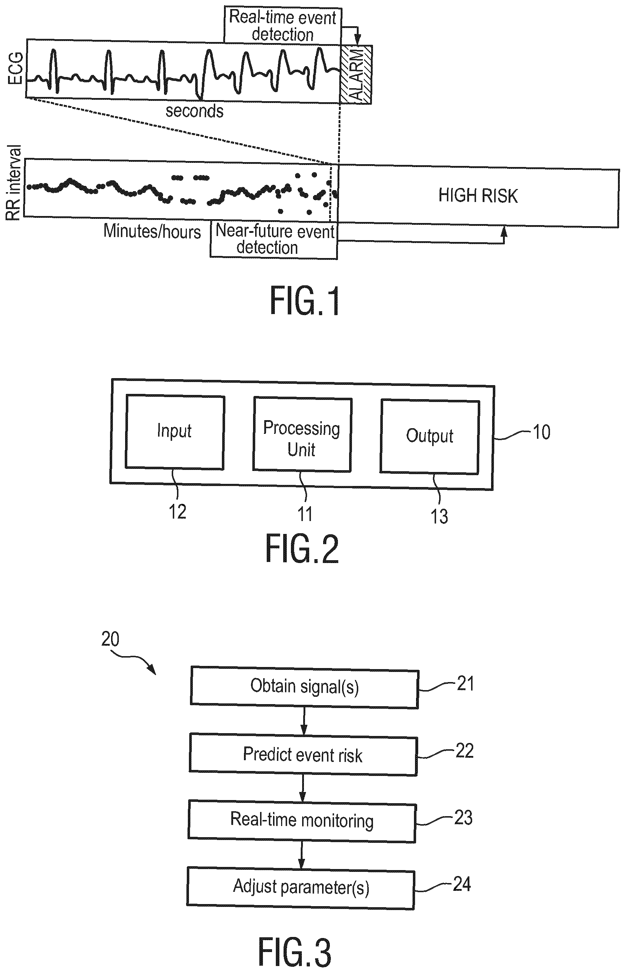

Predictive algorithms, i.e., algorithms or methods to determine an event risk of a future health adverse event of the patient, are generally designed to process windows for a longer duration, e.g. hours of physiological data, to identify the risk for adverse events in the following time, e.g. the following hours of monitoring. On the other hand, monitoring algorithms, i.e., algorithms or methods to real-time monitoring of a current health adverse event, process only a short duration, e.g. a few seconds, of physiological data to detect an adverse event and, e.g., generate alarms a few seconds after the onset of an event. Fig. 1 shows a diagram illustrating these differences in a typical ECG monitoring scenario. For real-time monitoring (upper diagram) an ECG is as physiological signal in this scenario, and for event risk prediction (lower diagram) a beat-to-beat interval (RR) signal is used as physiological signal in this scenario.

Generally, in an embodiment, for event risk determination one or more portions of the one or more obtained physiological signals of a duration in the range between 5 minutes and 10 hours, in particular the last minutes or hours of the one or more obtained physiological signals, are analyzed, and for real-time monitoring of the current health adverse event one or more portions of the one or more obtained physiological signals of a duration in the range between 10 seconds and 10 minutes, in particular the last seconds or minutes of the one or more obtained physiological signals, are analyzed. The physiological signal for both analyses may be identical or different (as in the example shown in Fig. 1).

Fig. 2 shows a schematic diagram of an embodiment of a patient monitoring device 10 for monitoring a patient according to the present invention. The patient monitoring device 10 comprises a processing unit 11 that is configured to process one or more physiological signals of the patient for real-time monitoring of a current health adverse event of the patient, e.g. for detecting cardiac arrhythmias like ventricular tachycardia, ventricular fibrillation, asystole and/or other life-threatening events. The processing unit may be any kind of means configured to process the physiological signal(s) accordingly and may be implemented in software and/or hardware, e.g. as a programmed processor or computer or app on a user device such as a smartphone, smartwatch, tablet, laptop, PC, workstation, bedside or portable patient monitor, etc.

For obtaining (i.e. receiving or retrieving) the one or more physiological signals, the patient monitoring device 10 may comprising a signal input unit 12. The signal input unit 12 may be directly coupled or connected to one or more physiological signal sensor(s) or may obtain (i.e. retrieve or receive) these signals from a storage, buffer, network, preprocessor or bus, etc. The signal input unit 12 may thus e.g. comprise a (wired or wireless) communication interface or data interface, such as a Bluetooth interface, Wi-Fi interface, LAN interface, HDMI interface, direct cable connection, or any other suitable interface allowing signal transfer to the patient monitoring device 10.

The patient monitoring device 10 may further comprise an output unit 13 configured to output the result of the real-time monitoring and, optionally, further information, such as the obtained physiological signal(s), any derived information (e.g. a risk score), the determined future risk for adverse event(s), alarm(s), recommendation(s) for treatment or actions, modifications in the configuration of the real-time algorithm for alarm generation, etc. The output unit 13 may comprise an interface that provides the information, e.g. transmits it to another device or provides it for retrieval by another device (e.g. a smartphone, computer, tablet, etc.). It may thus generally be any (wired or wireless) communication or data interface. In other embodiments, the output unit 13 comprises user interface for inputting and/or outputting information in visual and/or audible form, e.g. in text form, as image or diagram, as sound or spoken words, etc. The output unit 13 may e.g. comprise a user interface including one or more of a display, touchscreen, loudspeaker, computer monitor, screen of a smartphone and/or tablet, etc.

Fig. 3 shows a flowchart of a first embodiment of a patient monitoring method 20 according to the present invention. The steps of the method 20 may be carried out by the patient monitoring device 10, wherein the main steps of the method 20 are carried out by the processing unit 11. The method 20 may e.g. be implemented as computer program running on a computer or processor.

In a first step 21, one or more physiological signals of the patient are obtained. In a second step 22, an event risk of a future health adverse event of the patient is determined based on one or more of the obtained physiological signals. In a third step 23, real-time monitoring of a current health adverse event of the patient is carried out based on one or more of the obtained physiological signals using a monitoring algorithm having one or more adjustable configuration parameters. In a fourth step 24, one or more of the adjustable configuration parameters of the monitoring algorithm are adjusted based on the determined event risk.

Thus, according to the present invention the output of prediction algorithms is used to improve the way real-time detection in order to generate less false alarms during periods of low risk while increasing sensitivity in periods of high risk. This allows the real-time monitoring algorithm to be configured dynamically and flexibly and in a smart way in order to reduce clinical workload and preserve patient safety. For instance, a real-time monitoring algorithm for adverse event detection and real-time alarming may be provided with the ability of being configured in order to adjust its sensitivity and/or alarm behavior. These adjustments of the configurable parameter(s) of the monitoring algorithm may be carried out automatically. This solution may be applied in patient monitors, telemetry monitors and central stations which are thus enabled to process in real time ECG waveforms (and/or other physiological signals) and generate alarms for clinical intervention tailored to the expected risk for adverse events like lethal cardiac arrhythmias.

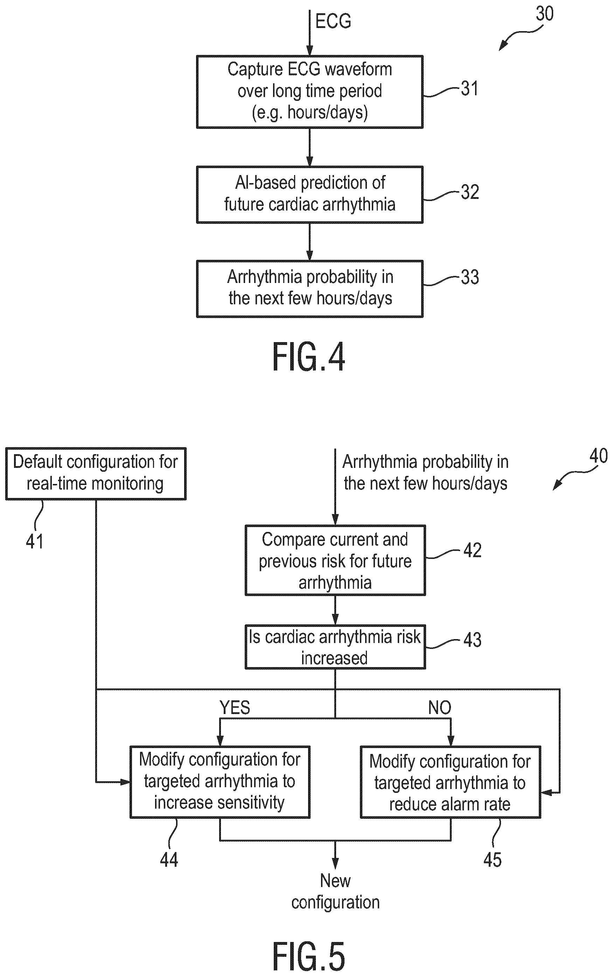

Fig. 4 shows a flowchart of an embodiment of a method 30 for near-future risk prediction for life threatening arrhythmia based on ECG from large windows of analysis and inference. The method 30 may be used, in an exemplary scenario, to implement step 22 in the embodiment of the patient monitoring method according to the present invention illustrated in Fig. 3.

The method 30 may use a prediction algorithm to process physiological waveforms which contain unexplored and complex clues related to the probability of future health adverse events (like cardiac arrhythmias). Targeted adverse events may be ventricular tachycardia, ventricular fibrillation, asystole, extreme bradycardia or extreme tachycardia, sudden cardiac arrest, atrial fibrillation, etc. Input data captured in step 31 may be the ECG measured by hospital monitoring equipment, but other input waveforms may be utilized such as PPG, invasive blood pressure, SpO2, respiration rate, etc. In addition, patient information like age, gender, ethnicity, BMI, treatments and other clinical information may be used, which may be obtained from a database (e.g. the patient's health record, a hospital database) or through input by the caregiver and/or the patient, e.g. at a user interface.

The prediction algorithm applied in step 32 may be an AI model including a sequence of convolutional and residual neural network components. The prediction algorithm can be composed of sub-parts designed to carry out specific tasks for waveform interpretation and the extraction of the context clues. Such sub-parts may be used to identify heartbeat location, heartbeat type, and the day-time patterns in the variability of such features. Input data would typically require large windows of waveform signal such as from 1 to 24 hours of data.

The output of the prediction algorithm provided in step 33 may be a risk score associated with the following hours or days of monitoring. These prediction windows may be designed according to the application and the related clinical interventions.

Fig. 5 shows a flowchart of an embodiment of a method 40 for adjusting/modifying one or more configuration parameters of a real-time monitoring algorithm based on the risk (e.g. probability) for onset of future arrhythmia. The method 40 may be used, in an exemplary scenario, to implement step 24 in the embodiment of the patient monitoring method according to the present invention illustrated in Fig. 3. This component may be called "Configuration modifier". It receives as input in step 41 the default configuration of the monitoring algorithm used to detect arrhythmia in real-time and generate alarms. The configuration of the monitoring algorithm, for this application scenario, may comprise one or more (preferably all) of the following configuration parameters:
- Amplitude of QRS detection threshold;
- Minimum event duration for arrhythmia like ventricular tachycardia;
- Sequence length of beats labelled as ventricular beats to identify a ventricular tachycardia;
- Alarm latching properties;
- Number of template families used to classify beats with new and different morphologies;
- Noise tolerance level; and
- Number of ECG waveform channels analyzed.

These configurations are particularly relevant for modifying configuration parameters in order to change sensitivity and/or alarm rate for ventricular tachycardia events. Different configuration parameters can be identified for other cardiac arrhythmias like ventricular fibrillation, asystole, or atrial fibrillation. In case the risk for arrhythmia has increased (step 43) based on a comparison of the current risk and the previous risk (step 42), the configuration can be modified to increase sensitivity for the monitoring algorithm (step 44). Alternatively, the configuration could be reverted to the default settings or modified to reduce alarm rate due to the low risk of future events (step 45).

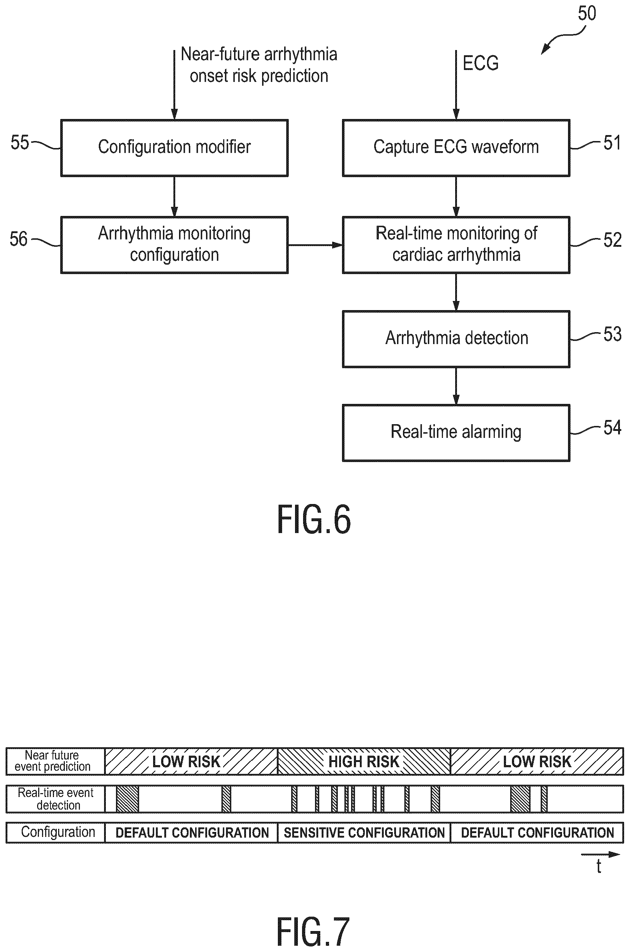

Fig. 6 shows a flowchart of a second embodiment of a patient monitoring method 50 according to the present invention. As explained above, the real-time monitoring is configurable, in particular to modify the detection sensitivity and/or alarming behavior. Input to the real-time monitoring step 52 are e.g. short window ECG signals captured from the patient in step 51. Outputs of the real-time monitoring step 52 are detected cardiac features (step 53), such as heartbeats, beat type, cardiac events, and alarms (step 54). The configuration modification method 40 explained above with reference to Fig. 5 may be used in step 55 to determine modifications of one or more configuration parameters (step 56) that are used in step 52 to adjust the configuration of the real-time monitoring. A user interface may be used to display the temporary change of configuration settings for the real-time monitoring in correspondence to a change in the risk profile for future cardiac arrhythmia (not shown in Fig. 6).

Fig. 7 shows a diagram illustrating the expected alarming behavior of the disclosed patient monitoring device. The first row shows the determined future risk of an adverse event (over time). The second row shows the result of real-time event detection, i.e., the alarming pattern. The third row shows the setting of the configuration of the real-time monitoring. As shown in Fig. 7, the alarming pattern (second row) and sensitivity (third row) by the real-time monitoring algorithm are influenced by the output from the prediction model as driven by a modification in the configuration settings. **In** other words, a change in configuration settings of the real-time monitoring algorithm is triggered by a modified risk for future adverse events. As a result, the real-time event alarming increases and then returns to default configurations once the risk has lowered.

In summary, the present invention may be applied in a monitoring scenario where patients are routinely monitored in a clinical setting to detect cardiac adverse events and provide surveillance. Cardiac arrhythmias like ventricular tachycardia, ventricular fibrillation and asystole are life-threatening events requiring prompt intervention by the nursing staff. Algorithms for real-time detection of cardiac arrhythmias are designed to generate alarms and not miss any true event. This causes the alarms to be often false and unnecessary leading to alarm fatigue. Artificial intelligence algorithms can be designed to interpret patient physiological waveforms and identify future risk for upcoming cardiac arrhythmias. The method and device according to the present invention improve the behaviour of real-time monitoring algorithms by utilizing information on future risk of upcoming lethal events to automatically tailor the alarming behaviour of patient monitoring equipment. This preserves patient safety in expected risky situations while achieving reduction of unnecessary alarms otherwise.

The present invention may be applied to cardiac monitoring, e.g. in hospital settings. Bedside monitors, telemetry monitors, central stations can benefit from this invention. Generally, the invention may be applied in all patient monitoring scenarios for real-time monitoring using one or more physiological signals of the patient.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Patient monitoring method for monitoring a patient, the method comprising:
- obtaining one or more physiological signals of the patient;
- determining an event risk of a future health adverse event of the patient based on one or more of the obtained physiological signals;
- real-time monitoring of a current health adverse event of the patient based on one or more of the obtained physiological signals using a monitoring algorithm having one or more adjustable configuration parameters; and
- adjusting one or more of the adjustable configuration parameters of the monitoring algorithm based on the determined event risk.

2. Patient monitoring method according to claim 1,
wherein one or more of the adjustable configuration parameters of the monitoring algorithm are adjusted to increase sensitivity of the monitoring algorithm if the determined event risk indicates an increased risk of a future health adverse event and/or to decrease sensitivity and increase precision of the monitoring algorithm if the determined event risk indicates a reduced risk of a future health adverse event.

3. Patient monitoring method according to claim 2,
wherein it is determined if the determined event risk indicates an increased or decreased risk of a future health adverse event based on one or more predetermined or settable risk thresholds or risk scores.

4. Patient monitoring method according to any one of the preceding claims,
wherein the adjustable configuration parameters of the monitoring algorithm include one or more of:
- amplitude of an event detection threshold;
- minimum event duration;
- one or more of sequence length, periodicity, number, interval of one or more event-related features in the one or more obtained physiological signals;
- one or more alarm latching properties;
- number and/or types of template families used for classification of one or more event-related features;
- noise tolerance level; and
- number of physiological signals analyzed;
- processing window of physiological signal analyzed, and
- operative point of a machine learning model configured to process one or more real-time signals physiological signals.

5. Patient monitoring method according to any one of the preceding claims,
wherein the one or more physiological signals include one or more of an ECG in one or more ECG channels, an inter-beat interval signal, a photoplethysmogram, blood pressure, SpO2, respiration rate, and body temperature.

6. Patient monitoring method according to any one of the preceding claims,
wherein the obtained physiological signal is an ECG and the adjustable configuration parameters of the monitoring algorithm include one or more of:
- amplitude of QRS detection threshold;
- minimum event duration for arrhythmia-like ventricular tachycardia;
- sequence length of beats labelled as ventricular beats indicating a ventricular tachycardia;
- one or more alarm latching properties;
- number and/or types of template families used for classification of beats with new and different morphologies;
- noise tolerance level; and
- number of ECG waveform channels analysed.

7. Patient monitoring method according to any one of the preceding claims,
wherein patient information, in particular one or more of age, weight, size, gender, ethnicity, BMI, medication, medical treatment, and medical history, is used in addition to the one or more obtained physiological signals to determine the event risk.

8. Patient monitoring method according to any one of the preceding claims,
wherein an AI-based prediction algorithm is used to to determine the event risk and/or to adjust one or more of the adjustable configuration parameters.

9. Patient monitoring method according to any one of the preceding claims,
wherein in case of a reduced event risk one or more of the adjustable configuration parameters of the monitoring algorithm are adjusted to use default configuration parameters, or to use previously used configuration parameters that have been previously used in case of a reduced event risk, or to maintain the currently used configuration parameters.

10. Patient monitoring method according to any one of the preceding claims,
wherein for determining the event risk one or more portions of the one or more obtained physiological signals of a duration in the range between 5 minutes and 10 hours, in particular the last minutes or hours of the one or more obtained physiological signals, are analyzed, and
wherein for real-time monitoring of the current health adverse event one or more portions of the one or more obtained physiological signals of a duration in the range between 10 seconds and 10 minutes, in particular the last seconds or minutes of the one or more obtained physiological signals, are analyzed.

11. Patient monitoring method according to any one of the preceding claims, further comprising generating and/or issuing one or more of:
- an alarm in case of detection of a current health adverse event;
- the one or more obtained physiological signals;
- the results of the detection of a current health adverse event and/or the determination of the event risk;
- a notification of the adjustment of one or more configuration parameters; and
- the one or more adjusted configuration parameters.

12. Patient monitoring device (10) for monitoring a patient, the device comprising a processing unit (11) configured to:
- obtain one or more physiological signals of the patient;
- determine an event risk of a future health adverse event of the patient based on one or more of the obtained physiological signals;
- real-time monitor of a current health adverse event of the patient based on one or more of the obtained physiological signals using a monitoring algorithm having one or more adjustable configuration parameters; and
- adjust one or more of the adjustable configuration parameters of the monitoring algorithm based on the determined event risk.

13. Patient monitoring device according to claim 12, further comprising a user interface (13) for inputting and/or outputting information, in particular including one or more of a display, touch screen, loudspeaker, computer monitor, screen of a smartphone and/or tablet.

14. Computer program comprising program code means for causing a computer to carry out the steps of the method according to any one of claims 1 to 11 when said computer program is carried out on the computer.
